# EUROPÄISCHE PATENTANMELDUNG

(11) **EP 1 041 856 A2**
(43) Veröffentlichungstag der Anmeldung: **04.10.2000**
(21) Anmeldenummer: 99122689.5
(22) Anmeldetag: 15.11.1999
(51) Int. Cl.: H04R 25/00

(54) **Vollimplantierbares Hörsystem mit telemetrischer Sensorprüfung**

(30) Priorität: 01.04.1999 DE 19914993
(71) Anmelder: IMPLEX Aktiengesellschaft Hearing Technology, 85737 Ismaning (DE)
(72) Erfinder: Leysieffer, Hans Dr.-Ing., 82024 Taufkirchen (DE)
(74) Vertreter: Schwan, Gerhard, Dipl.-Ing.

(57) **Zusammenfassung**

Vollimplantierbares Hörsystem zur Rehabilitation einer reinen Innenohrschwerhörigkeit oder einer kombinierten Schalleitungs- und Innenohrschwerhörigkeit, mit einem ein elektrisches Audiosignal abgebenden implantierbaren Sensor (10), einer in einem audiosignalverarbeitenden, elektronischen Hörsystempfad liegenden Signalbearbeitungs- und Verstärkungsseinheit (40, 50, 80, 140, 141), einem implantierbaren elektromechanischen Ausgangswandler (20) und einer Einheit (60) zur energetischen Versorgung des Implantatsystems. Das Hörsystem ist ferner versehen mit einer implantatseitigen Meßeinheit (13; 130, 140), die das elektrische Sensorsignal auf elektronischem Weg meßtechnisch erfaßt und elektronisch aufbereitet, und einer ebenfalls implantatseitig angeordneten drahtlosen Telernetrieeinheit (110), die das meßtechnisch erfaßte und elektronisch aufbereitete Sensorsignal nach außen zu einer externen Darstellungs- und/oder Bewertungseinheit (112) übermittelt.

## Beschreibung

Vollimplantierbare Hörsysteme zur Rehabilitation einer reinen Innenohrschwerhörigkeit oder einer kombinierten Schalleitungs- und Innenohrschwerhörigkeit mit mechanischer Stimulation des geschädigten Ohres befinden sich kurz vor der Markteinführung (Zeitschrift HNO 46:844-852, 10-1998, H.P. Zenner et al., "Erste Implantationen eines vollständig implantierbaren elektronischen Hörsystems bei Patienten mit Innenohrschwerhörigkeit"; US-A-5 277 694; US-A-5 788 711; US-A-5 814 095; US-A-5 554 096 und US-A-5 624 376). Solche Hörsysteme weisen grundsätzlich vier Funktionseinheiten auf, und zwar einen Sensor (Mikrofon), der den einfallenden Luftschall in ein elektrisches Signal umwandelt, eine elektronische Signalverarbeitungs- und Verstärkungseinheit, einen elektromechanischen Wandler, der die verstärkten und vorverarbeiteten Sensorsignale in mechanische Schwingungen umwandelt und diese über geeignete Koppelmechanismem dem geschädigten Mittel- und/oder Innenohr zuführt, und ein elektrisches Energieversorgungssystem, das die genannten Module versorgt. Weiterhin kann eine Einheit vorgesehen sein, die dem Implantat elektrische Nachladeenergie zur Verfügung stellt, wenn die implantatseitige Energieversorgungseinheit eine nachladbare (Sekundär-) Batterie enthält (US-A-5 279 292). Zweckmäßig ist auch eine Telemetrieeinheit vorgesehen, mit der patientenspezifische audiologische Daten drahtlos bidirektional übertragen beziehungsweise im Implantat programmiert und damit dauerhaft gespeichert werden können (Zeitschrift HNO 46:853-863, 10-1998, H. Leysieffer et al., "Ein vollständig implantierbares Hörsystem für Innenohrschwerhörige: TICA LZ 3001").

Insbesondere bei vollimplantierbaren Systemen entfällt die Sichtbarkeit des Systems, so daß neben den Vorteilen der hohen Klangqualität, des offenen Gehörgangs und der vollen Alltagstauglichkeit von einer hohen zukünftigen Patientenakzeptanz ausgegangen werden kann. Grundsätzlich wird bei diesen implantierbaren Hörsystemen als Ausgangssignal ein mechanischer, vibratorischer Stimulus verwendet, der das Mittelohr oder Innenohr direkt anregt. Die Ankopplung des mechanischen Reizes, der von einem elektromechanischen Wandler erzeugt wird, geschieht durch direkte mechanische Verbindung des schwingenden Wandlerelementes an die Ossikelkette oder ein Ossikel des Mittelohres oder an das Innenohr (US-A-5 941 814) oder durch eine Kraftkopplung über einen Luftspalt bei zum Beispiel elektromagnetischen Wandlern.

Die Wandlung des Luftschallsignals in ein elektrisches Signal, das in einer elektronischen Einheit weiterverarbeitet und verstärkt werden kann und dann so aufbereitet den implantierten, elektromechanischen Wandler zur Mittel- oder Innenohranregung ansteuert, erfolgt durch einen speziellen Sensor (Mikrofon), der bei vollimplantierbaren Hörsystemen subkutan, das heißt unter der geschlossenen Haut, positioniert wird. Als Implantationsort des Sensors werden akustisch und audiologisch vorteilhaft der Bereich des Gehörgangs, das Trommelfell selbst beziehungsweise der mit dem Trommelfell verwachsene Hammer oder andere Ossikel des Mittelohres gewählt. Der Sensor ist grundsätzlich ein mechanoelektrischer Wandler, dessen Eingangssignal eine mechanische Schwingung ist, die aus der akustischen Dichtewelle des eintreffenden Luftschalles resultiert. Solche implantierbaren, akustischen bzw. mechano-elektrischen Sensorsysteme sind in der wissenschaftlichen Literatur und in Patentschriften beschrieben (Zeitschrift HNO 45:816-827, 10-1997, H. Leysieffer et al., "Ein implantierbares Mikrofon für elektronische Hörimplantate", EP-A-0 831 673, US-A-5 814 095, EP-A-0 920 239, US-A-4 729 366, US-A-4 850 962 sowie WO 98/36711, WO 98/06237, US-A-5 859 916, WO 98/03035, WO 99/08481, WO 99/08475, WO 99/07436, WO 97/18689).

Die Funktion dieses schallwandelnden Sensors beziehungsweise Mikrofons bei einem vollimplantierbaren Hörsystem besteht darin, den externen, einfallenden Luftschall in ein elektrisches Signal umzuwandeln, das einer darauffolgenden elektronischen Signalverarbeitungs- und verstärkungseinheit zugeführt werden kann. Da bei einem Vollimplantat aus Biostabilitäts- und Hygienegründen grundsätzlich der Anspruch besteht, keine artifizielle und permanente Körperöffnung beziehungsweise Hautöffnung zu erzeugen, durch welche der zu wandelnde Schall einem Sensor beziehungsweise Mikrofon zugeführt werden kann, liegt biologisch aktives Gewebe zwischen dem Sensorelement und dem externen, schalltragenden Medium, das heißt Luft. Dieses Gewebe kann bei einem subkutan in die hintere Gehörgangswand implantierten Mikrofon ((Zeitschrift HNO 45:816-827, 10-1997, H. Leysieffer et al., "Ein implantierbares Mikrofon für elektronische Hörimplantate", EP-A-0 831 673, US-A-5 814 095, EP-A-0 920 239) die Gehörgangshaut sein, oder es kann im Fall einer direkten mechanischen Sensorkopplung an das Trommelfell beziehungsweise den Hammer in der Paukenhöhle eine knöcherne oder knorpelige Struktur sein (US-A-4 729 366, US-A-4 850 962, WO 98/36711, WO 98/06237, US-A-5 859 916, WO 98/03035, WO 99/08481, WO 99/08475, WO 99/07436, WO 97/18689). Bei diesen Arten der Sensor- beziehungsweise Mikrofonplazierung, die immer subkutan erfolgt, verbleibt die Unsicherheit einer langzeitstabilen, sicheren Kopplung, da diese unter anderem durch Nekrosenbildungen, vorübergehende oder permanente Serome, Gewebeneubildungen (zum Beispiel Bindegewebe), Luftdruckänderungen bei hermetisch dichten Druckwandlersensoren und sonstige externe und interne Einwirkungen beinflußt werden kann. Auch wenn diese Einflüsse durch geeignete Sensorauslegung minimiert werden können, verbleibt als variabler Faktor die interindividuelle Anatomie, die auf die Sensorübertragungsfunktion ebenfalls einwirken kann.

Die genaue Kenntnis dieser Sensorübertragungsfunktion, das heißt der frequenzabhängige Schalldruckübertragungsfaktor, der die Wandlung eines Schalldrucks in ein proportionales elektrisches Signal quantitativ beschreibt, ist für die individuelle Anpassung der audiologischen Hörsystemparameter, wie zum Beispiel die frequenzabhängige Verstärkung, von wesentlicher Bedeutung. Zwar kann intraoperativ mit geeigneten Meßmethoden eine a priori-Einschätzung der Sensorfunktionsdaten ermittelt werden, doch sind diese Daten sicherlich nicht identisch mit denen des eingeheilten, postoperativen Zustandes. Insbesondere sind auch Messungen der akustischen Richtcharakteristik des Sensors im implantierten Zustand von großem Interesse; solche Messungen sind intraoperativ grundsätzlich nicht möglich.

Der Erfindung liegt daher die Aufgabe zugrunde, ein vollimplantierbares Hörsystem zu schaffen, das individuell die objektive Messung der Sensorübertragungsfunktion im implantierten und postoperativen, eingeheilten Zustand ermöglicht.

Diese Aufgabe wird bei einem vollimplantierbaren Hörsystem zur Rehabilitation einer reinen Innenohrschwerhörigkeit oder einer kombinierten Schalleitungs- und Innenohrschwerhörigkeit, mit einem ein elektrisches Audiosignal abgebenden implantierbaren Sensor, einer in einem audiosignalverarbeitenden, elektronischen Hörsystempfad liegenden Signalbearbeitungs- und Verstärkungsseinheit, einem implantierbaren elektromechanischen Ausgangswandler und einer Einheit zur energetischen Versorgung des Implantatsystems, erfindungsgemäß dadurch gelöst, daß das Hörsystem implantatseitig mit einer Meßeinheit, die das elektrische Sensorsignal auf elektronischem Weg meßtechnisch erfaßt und elektronisch aufbereitet, und einer ebenfalls implantatseitig angeordneten drahtlosen Telemetrieeinheit ausgestattet ist, die das meßtechnisch erfaßte und elektronisch aufbereitete Sensorsignal nach außen zu einer externen Darstellungs- und/oder Bewertungseinheit übermittelt.

Die Bewertung des meßtechnisch erfaßten Sensorsignals kann extern erfolgen. Die implantatseitige Meßeinheit kann aber ebenfalls mit einer Bewertungseinheit versehen sein, um mindestens eine Vorbewertung bereits implantatseitig vorzunehmen. Beispielsweise kann es sich bei der implantatseitigen Bewertungseinheit um eine Einrichtung zur Spektralanalyse des Sensorsignals, wie eine Fast-Fourier-Transformation (FFT), handeln, deren Ergebnisdaten nach erfolgter Messung "offline" über die Telemetrieeinheit zur externen Darstellungs- und Bewertungseinheit übermittelt werden. Solche Lösungen sind insbesondere dann vorteilhaft realisierbar, wenn die Audiosignalverarbeitung des vollimplantierbaren Hörsystems auf digitaler Signalverarbeitung beruht, da bei dieser Realisierung das elektrische Sensorsignal direkt oder nach analoger Vorverstärkung in digitalisierter Fonn vorliegt und durch einen ebenfalls vorhandenen digitalen Signalprozessor solche zum Beispiel spektralen Bewertungsprozeduren auf einfache Weise durch Software-Algorithmen realisiert werden können, ohne daß ein zusätzlicher, implantatseitiger Hardware-Aufwand entsteht. Die implantatseitige Meßeinheit und die externe Bewertungseinheit können insbesondere zum Ermitteln des frequenzabhängigen Schalldruckübertragungsfaktors und/oder der akustischen Richtcharakteristik des Sensors im implantierten Zustand ausgelegt sein. Zweckmäßig kann das Hörsystem ferner mit einer elektronischen Einheit zum Anwählen und/oder Verändern von Meßfunktionen der implantatseitigen Meßeinheit versehen sein.

Das Hörsystem kann zum Übermitteln der von der Meßeinheit bereitgestellten Meßdaten in Echtzeit ausgelegt sein. Die Daten beispielsweise der Sensorübertragungsfunktion können, wenn implantatseitig entsprechende Speichermedien vorgesehen sind, auch zeitversetzt je nach Bedarf später ausgelesen werden.

Die elektronische Signalbearbeitungs- und Verstärkungsseinheit weist zweckmäßig einen dem Sensor nachgeschalteten Verstärker, eine mit dem Ausgangssignal des Verstärkers beaufschlagte audiologische Signalbearbeitungsstufe und einen dem elektromechanischen Ausgangswandler vorgeschalteten Treiberverstärker auf. Sie ist vorzugsweise mit einem digitalen Signalprozessor mit vorgeschalteten Analog-Digital-Wandler und nachgeschaltetem Digital-Analog-Wandler versehen.

Im Rahmen der Erfindung kann implantatseitig ein eigene Meßeinheit vorgesehen sein. Der Signalprozessor kann aber auch zusammen mit dem vorgeschalteten Analog-Digital-Wandler die implantatseitige Meßeinheit bilden.

Als implantierbarer elektromechanischer Ausgangswandler eignet sich insbesondere ein Wandler gemäß US-A-5 277 694, das heißt ein Wandler, bei dem eine Wand des Wandlergehäuses als schwingfähige Membran ausgeführt ist, die zusammen mit einer auf der Membraninnenseite aufgebrachten piezoelektrischen Keramikscheibe ein elektromechanisch aktives Heteromorph-Verbundelement darstellt.

Eine weitere für die vorliegenden Zwecke geeignete Wandlerbauart ist in der älteren EP-Patentanmeldung 98 121 495.0 beschrieben. Dabei handelt es sich um eine Wandleranordnung für teil- oder vollimplantierbare Hörgeräte zur direkten mechanischen Anregung des Mittel- oder Innenohres, die mit einem am Implantationsort mit Bezug auf den Schädel fixierbaren Gehäuse und einem mit Bezug auf das Gehäuse beweglichen, mechanisch steifen Koppelelement versehen ist, wobei in dem Gehäuse ein elektromechanischer Wandler untergebracht ist, mit dem sich das Koppelelement in Schwingungen versetzen läßt, die nach erfolgter Implantation der Wandleranordnung auf ein Mittelohr-Ossikel oder direkt auf das Innenohr übertragen werden. Der elektromechanische Wandler ist als Elektromagnetanordnung ausgebildet, die ein mit Bezug auf das Wandlergehäuse fixiertes Bauteil, insbesondere eine Ringspule, sowie ein schwingfähiges Bauteil, vorzugsweise in Form eines in eine Mittelöffnung der Ringspule eintauchenden Dauermagnetstifts, aufweist, welches mit dem Koppelelement derart in Verbindung steht, daß Schwingungen des schwingfähigen Bauteils auf das Koppelelement übertragen werden.

Vorteilhaft ist aber auch ein Wandler der in der älteren EP-Patentanmeldung 98 121 613.8 beschriebenen Art. Dabei handelt es sich um einen Wandler für teil- oder vollimplantierbare Hörgeräte zur direkten mechanischen Anregung des Mittel- oder Innenohres, der mit einem am Implantationsort fixierbaren Gehäuse und einem mit Bezug auf das Gehäuse beweglichen, mechanisch steifen Koppelelement versehen ist, wobei in dem Gehäuse ein piezoelektrisches Element untergebracht ist, mit dem sich das Koppelelement in Schwingungen versetzen läßt, die nach erfolgter Implantation des Wandlers auf ein Mittelohr-Ossikel oder direkt auf das Innenohr übertragen werden, und wobei in dem Gehäuse ferner eine Elektromagnetanordnung vorgesehen ist, die ein mit Bezug auf das Gehäuse fixiertes Bauteil sowie ein schwingfähiges Bauteil aufweist, welches mit dem Koppelelement derart in Verbindung steht, daß Schwingungen des schwingfähigen Bauteils auf das Koppelelement übertragen werden. Ein solcher Wandler hat den Vorteil, daß der Frequenzgang des Wandlers sowohl gegenüber rein piezoelektrischen als auch gegenüber rein elektromagnetischen Systemen verbessert werden kann, so daß ein adäquater Höreindruck bei ausreichendem Lautstärkepegel ermöglicht wird. Insbesondere kann ein weitgehend ebener Frequenzgang der Auslenkung des Koppelelements in einem weiten Frequenzband bei ausreichend hohen Stimulationspegeln und geringer Leistungsaufnahme verwirklicht werden.

Der Digital-Analog-Wandler und der Treiberverstärker können in einem Modul zusammengefaßt sein.

Der Signalprozessor ist vorzugsweise mit einem Datenspeicher zum Einspeichern von patientenspezifischen, audiologischen Anpaßparametem und/oder von Softwarealgorithmen für die Bewertung des elektrischen Sensorsignals ausgestattet.

Zum Steuern mindestens eines Teils der und vorzugsweise aller signalverarbeitenden und/oder -erzeugenden Stufen kann vorteihaft ein Mikrokontroller vorgesehen sein, der zweckmäßig einen Datenspeicher zum Einspeichern von patientenspezifischen, audiologischen Anpaßparametem und/oder von Parametern für die Meßfunktion der implantatseitigen Meßeinheit aufweist.

Der Signalprozessor kann aber auch selbst für des Steuern mindestens eines Teils der und vorzugsweise aller signalverarbeitenden und/oder -erzeugenden Stufen ausgelegt sein.

Die Telemetrieeinheit kann auch zur Dateneingabe in den Datenspeicher ausgelegt sein und mit einem externen Programmiersystem drahtlos oder drahtgebunden kommunizieren.

Wenn das Hörgerät vollimplantierbar ausgebildet ist, sind vorzugsweise die in dem elektronischen Hörsystempfad liegende Signalbearbeitungs- und Verstärkungsseinheit, die implantatseitige Meßeinheit und die Telemetrieeinheit als Elektronikmodul, zweckmäßig zusammen mit der Energieversorgungseinheit, in einem hermetisch dichten und biokompatiblen Implantatgehäuse untergebracht. Dabei ist vorteilhaft das Elektronikmodul über eine Implantatleitung mit einem in der hinteren Gehörgangswand subkutan implantierbaren Sensor und über eine implantierbare Leitung mit dem elektromechanischen Ausgangswandler verbunden. Diese Verbindung kann fest oder lösbar ausgebildet sein. Für eine lösbare Verbindung eignet sich insbesondere eine Steckverbindung, wie sie in US-A-5 755 743 im einzelnen erläutert ist. Eine solche Verbindungsanordnung weist mindestens einen ersten Kontakt, mindestens einen auf einem elastischen Körper gelagerten zweiten Kontakt und einen Verschlußmechanismus zum Ineingriffbringen der Stirnfläche des ersten Kontakts mit der Stirnfläche des zweiten Kontakts auf, wobei der erste Kontakt von mindestens einem Dichtungssteg umgeben ist, der bei einem Eingriff der Kontakte in den elastischen Körper eingepreßt ist und die Kontakte nach außen hin abdichtet.

Der Ausgangswandler ist vorzugsweise über ein Koppelelement mit einem Ossikel der Mittelohrkette zur Übertragung der ausgangseitigen mechanischen Wandlerschwingungen koppelbar. Dafür eignen sich insbesondere Lösungen der in US-A-5 277 694 und in US-A-5 941 814 beschriebenen Art. Dabei kann vorteilhaft ein aktiv schwingfähiges Teil des Ausgangswandlers mit einer Koppelstange mechanisch fest verbunden sein, die über ein Koppelelement an ein Teil der Ossikelkette angekoppelt wird. Zum Einstellen der Relativlage von Koppelstange und Koppelelement und zum Fixieren dieser Elemente in der eingestellten Relativlage ist vorzugsweise das Koppelelement mindestens im Fixierbereich hülsenförmig ausgebildet und mittels eines Crimpwerkzeugs plastisch kaltverformbar ist, während die Koppelstange mindestens im Fixierbereich stabförmig ausgebildet, mit rauher Oberfläche versehen und unter dem Einfluß der mittels des Crimpwerkzeugs ausgeübten Crimpkraft nicht plastisch kaltverformbar ist, wobei im fixierten Zustand der hülsenförmige Teil des Koppelelements durch die Crimpkraft kaltfließend verformt auf dem stabförmigen Teil der Koppelstange spielfrei und dauerhaft befestigt ist. Das von dem Ausgangswandler abliegende Ende der Koppelstange kann aber auch in eine Bohrung eines Teils der Ossikelkette eingesteckt und dort festgelegt sein.

Ferner kann der Ausgangswandler auch so ausgelegt sein, daß er über einen Luftspalt an die Ossikelkette oder das Innenohr ankoppelbar ist, wie dies im einzelnen in der US-A-5 015 225 beschrieben ist.

Zu einem vollimplantierbaren Hörgerät gehört in weiterer Ausgestaltung der Erfindung ein externes System zum transkutanen Übermitteln von patientenindividuellen Hörsystemdaten und von Programmierdaten für die implantatseitige Meßeinheit an die implantatseitige Telemetrieeinheit.

Als Energieversorgungseinheit kommen insbesondere eine Primärbatterie oder ein sekundäres, wiederaufladbares Element, das heißt ein nachladbarer Akkumulator, in Betracht. Im letztgenannten Fall ist vorzugsweise die Telemetrieeinheit zusätzlich als Energieempfangsschaltung zur implantatseitigen Bereitstellung von Nachladeenergie für die Energieversorgungseinheit ausgebildet, während das externe System zugleich als Ladegerät aufgebaut ist. Dafür eignen sich insbesondere ein Ladesystem der aus US-A-5 279 292 bekannten Art oder Anordnungen, wie sie in den älteren EP-Patentanmeldungen 98 121 496.8 und 98 121 498.4 beschrieben sind.

Zweckmäßig ist auch eine portable Fernbedienungseinheit zum Einstellen oder Verändern von Hörgeräte- und Audiometriefunktionen vorgesehen.

Nachfolgend sind vorteilhafte Ausführungsbeispiele der Erfindung anhand der Zeichnungen näher erläutert. Es zeigen:
- Fig. 1: ein Blockschaltbild eines erfindungsgemäßen vollimplantierbaren Hörsystems mit telemetrischer Sensorprüfung;
- Fign. 2 und 3: Blockschaltbilder von abgewandelten Ausführungsformen des vollimplantierbaren Hörsystems; und
- Fig. 4: eine schematische Darstellung eines vollimplantierbaren Hörsystems im implantierten Zustand.

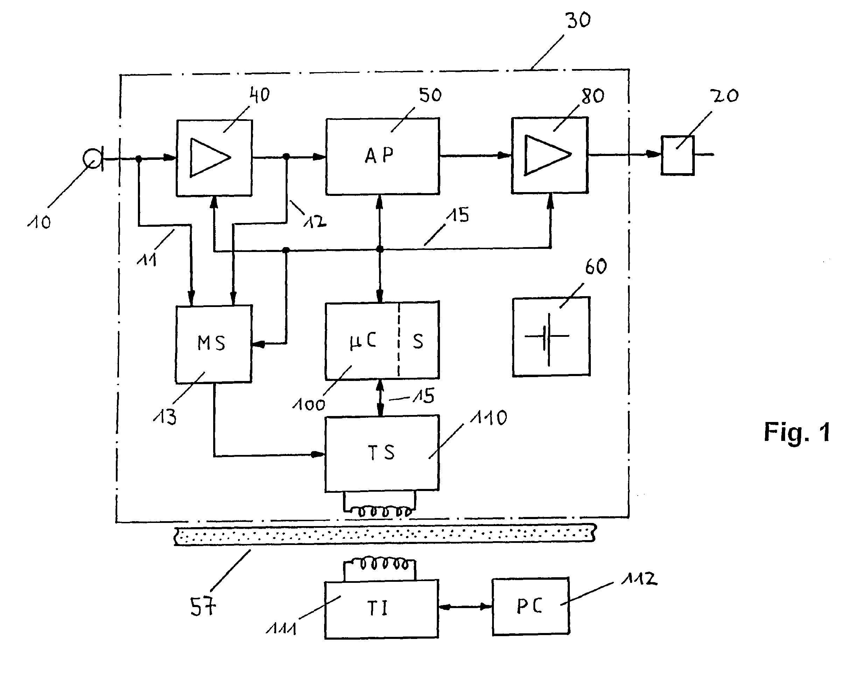

Das Hörimplantatsystem gemäß Fig. 1 weist einen Sensor 10 (Mikrofon) auf, mittels dessen das externe Schallsignal aufgenommen und in ein elektrisches Signal umgewandelt wird. Dieses Sensorsignal geht einem Implantatmodul 30 zu. In dem Modul 30 wird das elektrische Sensorsignal mittels eines Verstärkers 40 vorverstärkt. Dieses vorverstärkte Signal wird in einer audiologischen Signalbearbeitungsstufe 50 (AP: "Audio Prozessor") weiterbearbeitet. Diese Stufe kann alle bekannten, bei modernen Hörgeräten üblichen Komponenten wie Filterstufen, automatische Verstärkungsregelungen, Störsignalunterdrückungseinrichtungen und so weiter enthalten. Dieses bearbeitete Signal wird einem Treiberverstärker 80 zugeführt, der einen elektromechanischen Wandler 20 ansteuert. Der Wandler 20 stimuliert das geschädigte Innenohr durch direkte mechanische Kopplung an ein Mittelohrossikel oder über eine Luftspaltkopplung bei zum Beispiel elektromagnetischen, implantierbaren Wandlern. Die Signalbearbeitungskomponenten 40, 50, 80 und die Generatoren 90 werden durch einen Mikrokontroller 100 (µC) mit zugehörigem Datenspeicher (S) über einen uni- oder bidirektionalen Datenbus 15 gesteuert. In dem Speicherbereich S können insbesondere patientenspezifische, audiologische Anpaßparameter abgelegt sein. Diese individuellen, programmierbaren Daten werden dem Kontroller 100 über den Datenbus 15 durch ein Telemetriesystem 110 (T) zugeführt. Dieses Telemetriesystem 110 kommuniziert drahtlos durch die bei 57 angedeutete geschlossene Haut hindurch, beispielsweise wie in Fig. 1 dargestellt über eine induktive Spulenkopplung, bidirektional mit einem externen Telemetrieinterface 111 (TI). Das Telemetrieinterface 111 steht seinerseits in bidirektionaler Kommunikation mit einer Darstellungs- und/oder Bewertungseinheit 112, die vorteilhaft ein Computer (PC) mit entsprechender Bearbeitungs- und Darstellungssoftware sein kann.

Zusätzlich zu den oben beschriebenen, für eine Hörgerätefunktion notwendigen Modulen enthält das Implantatmodul 30 ein elektronische Meßsystem 13 (MS), dem das elektrische Sensorsignal über eine Leitung 11 direkt oder über eine Leitung 12 nach Vorverstärkung durch den Verstärker 40 zugeführt wird. Das Meßsystem 13 bereitet die Sensorinformation auf und leitet sie in das Telemetriesystem 110 weiter, so daß die Sensordaten nach außen über das Telemetrieinterface 111 zu der Darstellungseinheit 112 übertragen werden können. Das Meßsystem 13 wird ebenfalls über den Datenbus 15 des Implantatkontrollers 100 gesteuert, so daß je nach Bedarf das Sensorsignal auf der Leitung 11 oder das vorverstärkte Signal auf der Leitung 12 ausgewählt werden kann.

Das Meßsystem 13 kann insbesondere analoge, digitale und gemischt analoge und digitale Meßwertumformer, Bewertungsschaltungen und Modulationseinrichtungen beinhalten, die je nach Art des Telemetriesystems 110 entsprechend ausgewählt und optimiert sind. Die Parameter der Meßfunktionen des Meßsystems 13 können ebenfalls über den Datenbus 15 von dem Kontroller 100 angewählt beziehungsweise verändert werden. Die Anforderung einer Meßfunktion erfolgt über Steuerbefehle der externen Einheit 112 über das Telemetrieinterface 111. Diese Steuerbefehle lösen entsprechende Aktionen des implantatseitigen Kontrollers 100 aus.

Alle elektronischen Komponenten des Implantatsystems werden durch eine primäre oder eine nachladbare sekundäre Batterie 60 mit elektrischer Betriebsenergie versorgt. Der Sensor 10 und der elektromechanische Wandler 20 können an das Implantatmodul 30 fest oder gegebenenfalls über implantierbare Steckverbindungen angeschlossen sein.

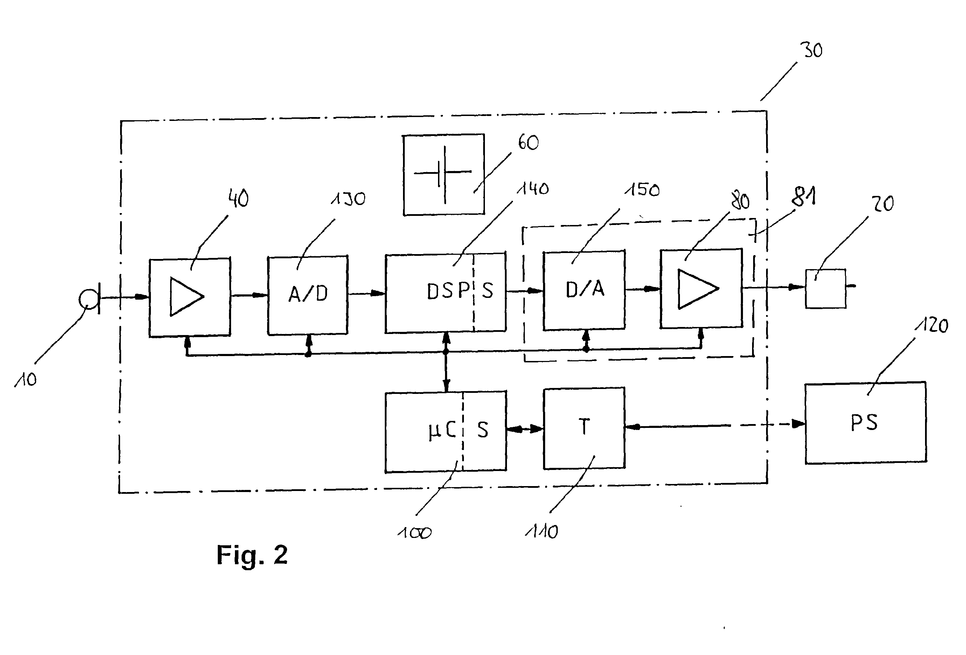

In Fig. 2 ist eine weitere Ausführungsform des elektronischen Implantatmoduls 30 dargestellt. Das Signal des Sensors 10 wird in dem Verstärker 40 vorverstärkt und mittels eines Analog-Digital-Wandlers 130 (A/D) in ein digitales Signal verwandelt, das einem digitalen Signalprozessor 140 (DSP) mit einem Datenspeicherbereich S zugeführt wird. Das digitale Ausgangssignal des Signalprozessors 140 wird in einem Digital-Analog-Wandler 150 (D/A) in ein analoges Signal rückgewandelt und über den Treiberverstärker 80 dem elektromechanischen Wandler 20 zugeführt.

Der Analog-Digital-Wandler 130 und der Signalprozessor 140 übernehmen im vorliegenden Fall zwei Aufgaben: einerseits wird, wie in volldigitalen Hörgeräten üblich, das Audiosignal den beschriebenen Signalbearbeitungsmethoden für eine Rehabilitation eines Innenohrschadens entsprechend aufbereitet und verarbeitet. Andererseits wird durch den Analog-Digital-Wandler 130 und den Signalprozessor 140 das Meßsystem 13 entsprechend Fig. 1 realisiert. Ein direkter Zugriff auf das Sensorsignal 11 wie in Fig. list hier jedoch nicht möglich, da das Sensorsignal vor der A/D-Wandlung vorverstärkt und tiefpaßgefiltert werden muß. Der notwendige Tiefpaß kann in dem Vorverstärker 40 realisiert sein.

Der Signalprozessor 140 übermittelt in einem beispielhaften Anwendungsfall die analogdigital gewandelten Sensorsignale an den Implantatkontroller 100, der diese über das Telemetriesystem 110 an die externe Darstellungs- und Bewertungseinheit übermittelt. In Fig. sind das Telemetrieinterface 111 und die Darstellungs- und Bewertungseinheit 112 aus Fig. 1 zusammenfassend als externes Programmiersystem 120 (PS) dargestellt. Bei entsprechender Auslegung der einzelnen Komponenten kann die telemetrische Übertragung der Sensordaten so schnell erfolgen, daß eine Quasi-Echtzeitmessung realisiert wird. So können beispielsweise Messungen der räumlichen Richtwirkung des im Patienten implantierten Sensors 10 durchgeführt werden. In einem weiteren beispielhaften Anwendungsfall kann der Signalprozessor 140 Softwarealgorithmen enthalten, die eine Bewertung des elektrischen Sensorsignals durchführen. Solche Bewertungen können beispielhaft zeitliche Mittelungen sein, um das Signal-Rausch-Verhältnis des Sensorausgangs bei akustischen Eingangssignalen geringen Pegels zu verbessern. Weiterhin können Algorithmen realisiert sein, die eine spektrale Bewertung des Sensorsignals bei breitbandigen akustischen Eingangsschallen (beispielsweise breitbandiges Testrauschen oder kurze Klicks, zum Beispiel akustische Dirac-Impluse) wie zum Beispiel eine Fast-Fourier-Transformation (FFT) ermöglichen, um so beispielsweise die frequenzabhängigen, akustischen Dämpfungseigenschaften des zwischen Luftschall und Implantatsensor gelegenen biologischen Mediums zu bestimmen. Weiterhin ergeben solche Spektralanalysen auch die Aspekte beziehungsweise Parameter der Sensor-Übertragungsfunktion, die durch die immer individuell unterschiedliche Anatomie des Patienten gegeben sind (beispielsweise Geometrieaspekte des äußeren Gehörgangs, Kopfform und dergleichen) oder durch zeitvariante Einflüsse wie zum Beispiel pathologische Veränderungen des Mittelohres (zum Beispiel otitis media). Die Ergebnisse dieser implantatinternen Messungen können als Meßdaten in dem Speicherbereich S des Signalprozessors 140 und/oder dem Speicherbereich des Implantatkontrollers 100 abgelegt sein, und sie werden bei Bedarf über das beschriebene Telemetriesystem 110 und das externe Programmier- und Bewertungssystem 120 ausgelesen.

Der D/A-Wandler 150 und der Treiberverstärker 80 können, wie in Fig. 2 durch einen Block 81 angedeutet ist, in einem Modul zusammengefaßt sein. Dies ist insbesondere in dem Fall vorzuziehen, in dem als Wandler 20 ein elektromagnetisches System verwendet wird und in dem Ausgangssignal des Signalprozessors 140 die Signalinformation durch Pulsweitenmodulation enthalten ist, so daß die für die Rückwandlung in ein Analogsignal erforderliche zeitliche Integration direkt von dem Wandler 20 übernommen wird.

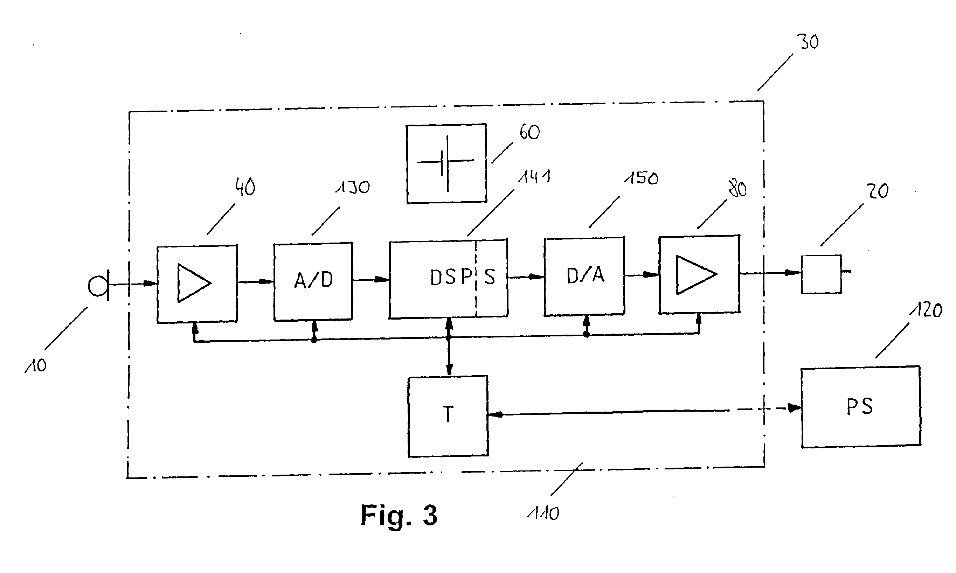

Die Ausführungsform gemäß Fig. 3 unterscheidet sich von derjenigen der Fig.2 im wesentlichen nur dadurch, daß ein Signalprozessor 141 vorgesehen ist, der auch die Funktionen des Mikrokontrollers 100 gemäß Fig. 2 übernimmt. Dabei werden die patientenspezifischen Daten der Audiosignalbearbeitung sowie die beschriebenen Ergebnisdaten des Meßsystemalgorithmus des Sensorsignals in dem Datenspeicherbereich S des Signalprozessors 141 abgelegt.

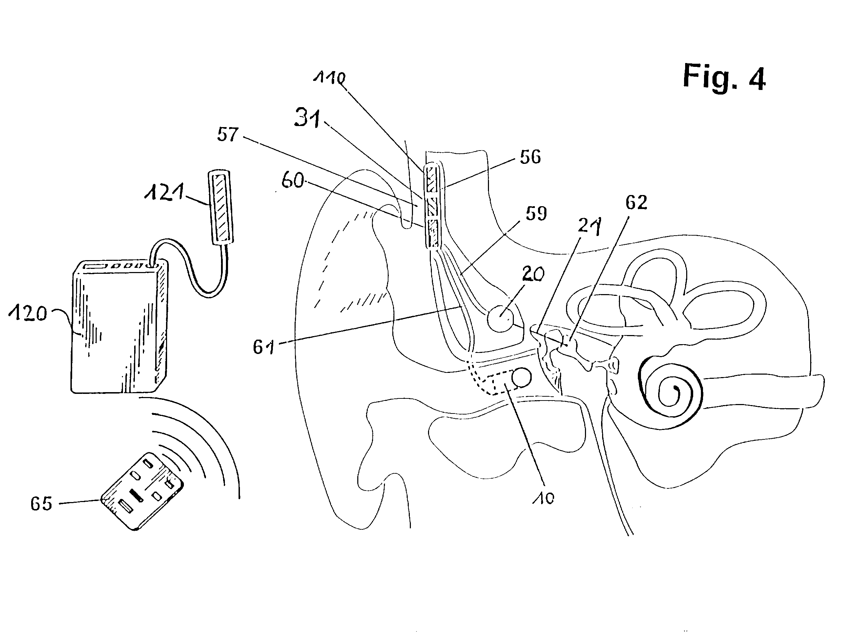

In Fig. 4 ist eine mögliche Ausführungsform des vollimplantierbaren Hörsystems mit telemetrischer Sensorprüfung entsprechend Fig. 1, Fig. 2 oder Fig. 3 schematisch dargestellt. In einem hermetisch dichten und biokompatiblen Implantatgehäuse 56 ist ein Elektronikmodul 31 (ohne Batterie dargestellt) untergebracht, das bis auf das Fehlen der Batterie dem Modul 30 der Figuren 1, 2 und 3 entspricht. Weiterhin enthält das Gehäuse 56 die Batterie 60 zur elektrischen Versorgung des Implantates sowie die Telemetrieeinrichtung 110. Der Sensor (Mikrofon) 10 ist vorzugsweise in der aus US-A-5 814 095 bekannten Weise, gegebenenfalls unter Verwendung des in EP-A-0 920 239 beschriebenen Fixationselements, in der hinteren Gehörgangswand subkutan implantiert. Der Sensor 10 nimmt den Schall auf und wandelt ihn in ein elektrisches Signal um, das über eine Implantatleitung 61 dem Elektronikmodul 31 in dem Gehäuse 56 zugeführt wird. Das audiologisch bearbeitete und verstärkte Signal gelangt über eine implantierbare Leitung 59 zu dem elektromechanischen Wandler 20. Dieser Wandler 20 ist im vorliegenden Beispiel als direktgekoppeltes System dargestellt, das heißt, die ausgangsseitigen mechanischen Schwingungen des Wandlers 20 werden über ein geeignetes Koppelelement 21 direkt an ein Ossikel der Mittelohrkette gekoppelt, im dargestellten Fall an den Amboß 62. Vorzugsweise geschieht dies in der an sich aus US-A-5 277 694 und US-A-5 788 711 bekannten Weise. Die dort eingekoppelten Wandlerschwingungen gelangen über die Ossikelkette zum Innenohr und rufen dort den entsprechenden Höreindruck hervor.

Weiterhin ist in Fig. 4 das externe Programmier-, Darstellungs- und Bewertungssystem 120 dargestellt, mit dem wie beschrieben die patientenindividuellen Hörgerätedaten transkutan programmiert und gelesen werden sowie die implantatintern gemessenen Sensordaten übermittelt werden. Dazu dient ein Sende- und Lesekopf 121, der zur bidirektionalen Datenübermittlung über das Implantat gebracht wird und zum Beispiel auf induktivem Weg die Daten transferiert. Ist die Batterie 60 im Implantatgehäuse 56 ein sekundäres, wiederaufladbares Element, kann die Einheit 110 auch eine Energieempfangsschaltung zur implantatseitigen Bereitsstellung von Nachladeenergie sein. Dann ist das externe System 120 mit dem Sendekopf 121 ein zum Beispiel portables, drahtloses Ladegerät. Dabei können vorzugsweise Anordnungen vorgesehen werden, wie sie an sich aus US-A-5 279 292 bekannt beziehungsweise in den älteren EP-Patentanmeldungen 98 121 496.8 und 98 121 498.4 näher erläutert sind. Weiter ist eine portable Fembedienungseinheit 65 dargestellt, mit der der Patient wesentliche Hörgerätefunktionen einstellen beziehungsweise verändern kann.

## Patentansprüche

1. Vollimplantierbares Hörsystem zur Rehabilitation einer reinen Innenohrschwerhörigkeit oder einer kombinierten Schalleitungs- und Innenohrschwerhörigkeit, mit einem ein elektrisches Audiosignal abgebenden implantierbaren Sensor (10), einer in einem audiosignalverarbeitenden, elektronischen Hörsystempfad liegenden Signalbearbeitungs- und Verstärkungsseinheit (40, 50, 80, 140, 141), einem implantierbaren elektromechanischen Ausgangswandler (20) und einer Einheit (60) zur energetischen Versorgung des Implantatsystems, **gekennzeichnet durch** eine implantatseitige Meßeinheit (13; 130, 140, 141), die das elektrische Sensorsignal auf elektronischem Weg meßtechnisch erfaßt und elektronisch aufbereitet, und eine ebenfalls implantatseitig angeordnete drahtlose Telemetrieeinheit (110), die das meßtechnisch erfaßte und elektronisch aufbereitete Sensorsignal nach außen zu einer externen Darstellungs- und/oder Bewertungseinheit (112) übermittelt.

2. Hörsystem nach Anspruch 1, dadurch gekennzeichnet, daß die implantatseitige Meßeinheit (13; 130, 140, 141) eine Bewertungseinheit beinhaltet.

3. Hörsystem nach Anspruch 1 oder 2, dadurch gekennzeichnet, daß die implantatseitige Meßeinheit (13; 130, 140, 141) und die externe Bewertungseinheit (112) zum Ermitteln des frequenzabhängigen Schalldruckübertragungsfaktors und/oder der akustischen Richtcharakteristik des Sensors (10) im implantierten Zustand ausgelegt sind.

4. Hörsystem nach einem der vorhergehenden Ansprüche, gekennzeichnet durch eine elektronische Einheit (100) zum Anwählen und/oder Verändern von Meßfunktionen der implantatseitigen Meßeinheit (13; 130, 140, 141).

5. Hörsystem nach einem der vorhergehenden Ansprüche, dadurch gekennzeichnet, daß die Telemetrieeinheit (110) zum Übermitteln der von der Meßeinheit (13; 130, 140, 141) bereitgestellten Meßdaten in Echtzeit ausgelegt ist.

6. Hörsystem nach einem der Ansprüche 1 bis 4, dadurch gekennzeichnet, daß es für ein implantatseitiges Speichern der von der Meßeinheit (13; 130, 140, 141) bereitgestellten Meßdaten ausgelegt ist und die gespeicherten Meßdaten nach Bedarf zeitversetzt über die Telemetrieeinheit (110) auslesbar sind.

7. Hörsystem nach einem der vorhergehenden Ansprüche, dadurch gekennzeichnet, daß die elektronische Signalbearbeitungs- und Verstärkungsseinheit (40, 50, 80, 140, 141) einen dem Sensor (10) nachgeschalteten Verstärker (40), eine mit dem Ausgangssignal des Verstärkers (40) beaufschlagte audiologische Signalbearbeitungsstufe (50, 140, 141) und einen dem elektromechanischen Ausgangswandler (20) vorgeschalteten Treiberverstärker (80) aufweist.

8. Hörsystem nach einem der vorhergehenden Ansprüche, dadurch gekennzeichnet, daß die elektronische Signalbearbeitungs- und Verstärkungsseinheit einen digitalen Signalprozessor (140, 141) mit vorgeschalteten Analog-Digital-Wandler (130) und nachgeschaltetem Digital-Analog-Wandler (150) aufweist.

9. Hörsystem nach Anspruch 8, dadurch gekennzeichnet, daß der Signalprozessor (140, 141) zusammen mit dem vorgeschalteten Analog-Digital-Wandler (130) die implantatseitige Meßeinheit bildet.

10. Hörsystem nach Ansprüchen 7 und 9, dadurch gekennzeichnet, daß der dem Signalprozessor (140) nachgeschaltete Digital-Analog-Wandler (150) und der Treiberverstärker (80) in einem Modul (81) zusammengefaßt sind.

11. Hörsystem nach einem der Ansprüche 8 bis 10, dadurch gekennzeichnet, daß der Signalprozessor (140, 141) einen Datenspeicher (S) zum Einspeichern von patientenspezifischen, audiologischen Anpaßparametem und/oder von Softwarealgorithmen für die Bewertung des elektrischen Sensorsignals aufweist.

12. Hörsystem nach einem der vorhergehenden Ansprüche, dadurch gekennzeichnet, daß mindestens ein Teil der signalverarbeitenden und/oder ―erzeugenden Stufen (40, 50, 80, 130, 140, 141, 150) mittels eines Mikrokontrollers (100) gesteuert ist.

13. Hörsystem nach Anspruch 12, dadurch gekennzeichnet, daß der Mikrokontroller (100) einen Datenspeicher (S) zum Einspeichern von patientenspezifischen, audiologischen Anpaßparametern und/oder von Parametern für die Meßfunktion der implantatseitigen Meßeinheit (13; 130, 140, 141) aufweist.

14. Hörsystem nach einem der Ansprüche 8 bis 13, dadurch gekennzeichnet, daß der Signalprozessor (141) selbst für das Steuern mindestens eines Teils der signalverarbeitenden und/oder ―erzeugenden Stufen (40, 80, 130, 150) ausgelegt ist.

15. Hörsystem nach Anspruch 11 oder Anspruch 13, dadurch gekennzeichnet, daß die Telemetrieeinheit (110) zur Dateneingabe in den Datenspeicher (S) ausgelegt ist.

16. Hörsystem nach Anspruch 15, gekennzeichnet durch ein mit der Telemetrieeinheit (110) drahtlos oder drahtgebunden kommunizierendes externes Programmiersystem (120).

17. Hörsystem nach Anspruch 15 oder 16, dadurch gekennzeichnet, daß das Gerät vollimplantierbar ausgebildet ist und die in dem elektronischen Hörsystempfad liegende Signalbearbeitungs- und Verstärkungsseinheit (40, 50, 80, 140, 141), die implantatseitige Meßeinheit (13; 130, 140, 141) sowie die Telemetrieeinheit (110) als Elektronikmodul (31), vorzugsweise zusammen mit der Energieversorgungseinheit (60), in einem hermetisch dichten und biokompatiblen Implantatgehäuse (56) untergebracht sind.

18. Hörsystem nach Anspruch 17, dadurch gekennzeichnet, daß das Elektronikmodul (31) über eine Implantatleitung (61) mit einem in der hinteren Gehörgangswand subkutan implantierbaren Sensor (20) verbunden ist.

19. Hörsystem nach Anspruch 17 oder 18, dadurch gekennzeichnet, daß das Elektronikmodul (31) über eine implantierbare Leitung (59) mit dem elektromechanischen Ausgangswandler (20) verbunden ist.

20. Hörsystem nach einem der vorhergehenden Ansprüche, dadurch gekennzeichnet, daß der Ausgangswandler (20) über ein Koppelelement (21) mit einem Ossikel der Mittelohrkette zur Übertragung der ausgangseitigen mechanischen Wandlerschwingungen koppelbar ist.

21. Hörsystem nach einem der Ansprüche 1 bis 19, dadurch gekennzeichnet, daß der Ausgangswandler (20) derart ausgelegt ist, daß er über einen Luftspalt an die Ossikelkette oder das Innenohr ankoppelbar ist.

22. Hörsystem nach einem der Ansprüche 17 bis 21, gekennzeichnet durch ein externes System (120) zum transkutanen Übermitteln von patientenindividuellen Hörsystemdaten und von Programmierdaten für die implantatseitige Meßeinheit (13; 130, 140, 141) an die implantatseitige Telemetrieeinheit (110).

23. Hörsystem nach Anspruch 22, dadurch gekennzeichnet, daß als Energieversorgungseinheit (60) ein sekundäres, wiederaufladbares Element vorgesehen ist, die Telemetrieeinheit (110) zusätzlich als Energieempfangsschaltung zur implantatseitigen Bereitstellung von Nachladeenergie für die Energieversorgungseinheit ausgebildet ist und das externe System (120) zugleich als Ladegerät aufgebaut ist.

24. Hörsystem nach einem der Ansprüche 17 bis 23, gekennzeichnet durch eine portable Fernbedienungseinheit (65) zum Einstellen oder Verändern von Hörsystem- und Audiometriefünktionen.
